# EUROPÄISCHE PATENTSCHRIFT

(11) **EP 1 529 028 B1**
(45) Veröffentlichungstag und Bekanntmachung des Hinweises auf die Patenterteilung: **25.10.2006**
(21) Anmeldenummer: 03794858.5
(22) Anmeldetag: 08.08.2003
(51) Int. Cl.: C07C 209/86, C07C 211/36

(54) **DESTILLATIVE VERFAHREN ZUR HERSTELLUNG VON IPDA MIT EINEM HOHEN CIS/TRANS-ISOMERENVERHÄLTNIS**
METHOD FOR PRODUCING IPDA WITH A HIGH CIS/TRANS ISOMER RATIO BY DISTILLATION
PROCEDE DE PRODUCTION PAR DISTILLATION D'IPDA AYANT UN RAPPORT CIS/TRANS-ISOMERE ELEVE

(30) Priorität: 09.08.2002 DE 10236674
(43) Veröffentlichungstag der Anmeldung: 11.05.2005
(73) Patentinhaber: BASF Aktiengesellschaft, 67056 Ludwigshafen (DE)
(72) Erfinder: FUNKE, Frank, 67067 Ludwigshafen (DE); HILL, Thomas, 67071 Ludwigshafen (DE); VON WATZDORF, Jobst, Rüdiger, 68163 Mannheim (DE); MATTMANN, Wolfgang, 67117 Limburgerhof (DE); HARDER, Wolfgang, 69469 Weinheim (DE); HENKES, Erhard, 64683 Einhausen (DE); LITTMANN, Gerd, 69469 Weinheim (DE); JULIUS, Manfred, 67117 Limburgerhof (DE)
(74) Vertreter: Isenbruck, Günter
(86) Internationale Anmeldenummer: PCT/EP2003/008831
(87) Internationale Veröffentlichungsnummer: WO 2004/024668

(56) Entgegenhaltungen:
- DE-A- 4 211 454
- DE-A- 4 343 891

## Beschreibung

Die Erfindung betrifft destillative Verfahren zur Herstellung von 3-Aminomethyl-3,5,5-trimethylcyclohexylamin (Isophorondiamin, IPDA) mit einem hohen cis/trans-Isomerenverhältnis.

IPDA wird als Ausgangsprodukt zur Herstellung von Isophorondiisocyanat (IPDI), einer Isocyanatkomponente für Polyurethansysteme, als Aminkomponente für Polyamide und als Härter für Epoxidharze verwendet. IPDA wird üblicherweise aus 3-Cyano-3,5,5-trimethylcyclohexanon (Isophoronnitril, IPN) hergestellt, wobei in Gegenwart von Ammoniak, Wasserstoff und üblichen Hydrierkatalysatoren die Carbonylgruppe in eine Aminogruppe und die Nitrilgruppe in eine Aminomethylgruppe überführt werden. Man erhält Gemische aus cis-IPDA und trans-IPDA. Beide Isomere weisen unterschiedliche Reaktivitäten auf, was für die vorgesehene technische Anwendung von Bedeutung ist. Gemäß DE-A 42 11 454 werden durch Verwendung eines IPDA-Isomerengemischs, bestehend aus über 40% des trans-Isomeren und unter 60% des cis-Isomeren, als Reaktionskomponente in Polyadditionsharzen, wie insbesondere Epoxidharzen, sowohl die Topfzeit verlängert als auch die maximale Härtungstemperatur erniedrigt. Zur Erzielung einer möglichst hohen Reaktionsgeschwindigkeit werden umgekehrt IPDA-Isomerengemische bevorzugt, welche einen möglichst hohen Anteil an dem cis-Isomeren (≥ 70%) aufweisen. Kommerziell erhältliches IPDA besitzt deshalb ein cis/trans-Isomerenverhältnis von 75/25.

Unterschiedliche Verfahren zur Erzielung eines hohen cis/trans- bzw. eines hohen trans/cis-Verhältnisses sind aus dem Stand der Technik bereits bekannt.

Gemäß der DE-A 43 43 890 erfolgt die aminierende Hydrierung des IPN zum IPDA, indem man ein Gemisch aus IPN, Ammoniak und einem C₁-C₃-Alkohol in Gegenwart von Wasserstoff über einen mit einem Cobalt- und/oder Ruthenium-Festbettkatalysator ausgestatteten Rieselbettreaktor bei 3 bis 8 MPa und einer Temperatur von 40 bis 150°C, vorzugsweise 90 bis 130°C, rieseln lässt und das Reaktionsgemisch zur Abtrennung von NH₃, H₂O und Nebenprodukten destillativ aufarbeitet. Bei Verwendung eines Ru-Trägerkatalysators werden hohe cis/trans-Isomerenverhältnisse von 84/16 (Gesamtausbeute an IPDA: 81 %) erzielt.

Die DE-A 43 43 891 beschreibt ein Verfahren zur Herstellung von IPDA, wobei IPN in Gegenwart von Ammoniak und einem Suspensions- oder Festbett-Hydrierkatalysator aus der Reihe der Cobalt-, Nickel- und Edelmetallkatalysatoren mit Wasserstoff bei einem Druck von 3 bis 20 MPa und einer Temperatur bis zu 150°C umgesetzt und das erhaltene Reaktionsgemisch destillativ aufgearbeitet wird. Die Reaktion wird zweistufig durchgeführt, wobei genau definierte Temperaturbereiche für die einzelnen Stufen eingehalten werden müssen. Ein cis/trans-Isomerenverhältnis von 80/20 lässt sich in einer Gesamtausbeute an IPDA von 91,9% erzielen.

In dem Verfahren der EP-A 0 926 130 wird die Hydrierung in Gegenwart einer Säure an Katalysatoren durchgeführt, die Kupfer und/oder ein Metall der achten Nebengruppe des Periodensystems enthalten. Es werden sowohl Lewis- als auch Brönstedt-Säuren eingesetzt; bevorzugt wird 2-Ethylhexansäure verwendet. Die Säure-Zugabe bewirkt eine Erhöhung des cis/trans-Isomerenverhältnisses. Die cis/trans-Isomerenverhältnisse sind im allgemeinen ≥ 70/30 bei einer Gesamtausbeute an IPDA ≥ 90%.

Das Verfahren der EP-B 0 729 937 ist dadurch charakterisiert, dass das Verfahren in drei räumlich voneinander getrennten Reaktionsräumen durchgeführt wird, wobei cobalt-, nickel-, ruthenium- und/oder andere edelmetallhaltige Katalysatoren eingesetzt werden. Vor dem zweiten Reaktor wird wäßrige NaOH-Lösung zudosiert, wodurch die Bildung von ringförmigen Nebenprodukten wie 1,3,3-Trimethyl-6-azabicyclo[3,2,1]octan verringert wird.

In dem Verfahren der prioritätsälteren, nicht vorveröffentlichten DE-A 101 42 635.6 wird IPDA mit einem cis/trans-Isomerenverhältnis von mindestens 70/30 ausgehend von IPN gewonnen, indem in dem Hydrierungsschritt ein Hydrierkatalysator mit einem Alkalimetallgehalt ≤ 0,03 Gew.-% - berechnet als Alkalimetalloxid - eingesetzt wird.

Nachteilig an den bekannten Verfahren zur Herstellung von IPDA mit hohem cis-Anteil ist die aufwendige Herstellung der verwendeten Katalysatoren. Zudem unterliegen diese Katalysatoren im allgemeinen einer Alterung, wodurch ihre katalytische Aktivität im Laufe der Zeit abnimmt. Um dies auszugleichen, wird meist die Reaktionstemperatur erhöht, was jedoch zu einer Verschlechterung des cis/trans-Isomerenverhältnisses und der Selektivität und damit zu einer Zunahme der Bildung von Nebenprodukten führt. Die meisten, aus dem Stand der Technik bekannten Verfahren zeichnen sich zudem durch eine aufwendige Reaktionsführung aus.

Ein Verfahren zur Herstellung von Isophorondiamin mit einem hohen trans/cis-Isomerenverhältnis ist der DE-A 42 11 454 zu entnehmen. Hierbei wird trans-Isophorondiamin aus Isophoronnitril über das Isophoronnitrilazin hergestellt. Es wird auch beschrieben, dass sich das trans-Isophorondiamin durch Destillation von kommerziell erhältlichem cis/trans-Isomerengemisch gewinnen lassen würde. Da das cis-Isomer jedoch als Hauptprodukt anfällt, ist dieses Verfahren nicht wirtschaftlich. Über die in der Destillation eingesetzten Vorrichtungen werden keine Angaben gemacht.

Aufgabe der Erfindung ist es, ein Verfahren zur Herstellung von Isophorondiamin (IPDA) mit einem cis/trans-Isomerenverhältnis von mindestens 73/27 bereitzustellen, durch das die Nachteile des Standes der Technik vermieden werden.

Die Aufgabe wird erfindungsgemäß gelöst durch ein Verfahren zur Gewinnung von 3-Aminomethyl-3,5,5-trimethylcyclohexylamin (Isophorondiamin, IPDA) mit einem cis/trans-Isomerenverhältnis von mindestens 73/27 durch fraktionierte Destillation, welches folgende Schritte enthält:
a) Bereitstellen von IPDA mit einem cis/trans-Isomerenverhältnis < 73/27;
b) Einleiten von IPDA in den mittleren Bereich einer Destillationskolonne mit Einbauten und Destillieren des IPDA in dieser Destillationskolonne bei einer Sumpftemperaturen von 170 bis 250°C, und eines Kopftemperates von 10 bis 90°C und einem Druck von 20 bis 200 mbar;
c) gegebenenfalls weiteres Aufreinigen des durch Schritt b) erhaltenen IPDA durch Destillieren in mindestens einer weiteren Kolonne;
wobei durch die Schritte b) und c) eine Auftrennung des in Schritt a) eingesetzten IPDA in mindestens fünf Fraktionen ia) bis iv) erfolgt:
ia) der organische Anteil einer Fraktion niedriger als trans-IPDA siedender Verunreinigungen,
ib) der wäßrige Anteil einer Fraktion niedriger als trans-IPDA siedender Verunreinigungen,
ii) eine Fraktion höher als cis-IPDA siedender Verunreinigungen,
iii) eine IPDA-Fraktion mit einem cis/trans-Isomerenverhältnis ≥ 73/27 und
iv) eine IPDA-Fraktion mit einem cis/trans-Isomerenverhältnis ≤ 66/34.

Durch das erfindungsgemäße Verfahren kann prinzipiell - ausgehend von IPDA mit jedem beliebigen cis/trans-Isomerenverhältnis - IPDA mit einem definierten, konstanten cis/trans-Isomerenverhältnis gewonnen werden, wobei ein hoher anwendungsgemäß spezifizierbarer cis-Gehalt definiert eingestellt werden kann. Das Verfahren ist damit weitgehend unabhängig von dem vorgegebenen cis/trans-Isomerenverhältnis und bleibt auch wirtschaftlich, wenn durch eine Alterung des Katalysators und einer damit verbundenen Erhöhung der Reak-tionstemperatur der cis-Anteil im IPDA im Laufe der Zeit sinkt. Besonders wirtschaftlich ist das erfindungsgemäße Verfahren, wenn IPDA mit einem cis/trans-Isomerenverhältnis ≥ 73/27 aus IPDA mit einem cis/trans-Isomerenverhältnis < 73/27 gewonnen wird.

Bei Fraktion ib) handelt es sich um in dem erfindungsgemäßen Verfahren anfallendes behandlungsbedürftiges Abwasser. Der Anteil von IPDA in der durch die Schritte b) und c) erhaltenen Fraktion ib) ist im allgemeinen ≤ 2 Gew.-%, bevorzugt ≤ 1000 ppm, besonders bevorzugt ≤ 200 ppm, - bezogen auf das Gesamtgewicht der Fraktion ib).

Die einzelnen Schritte des Verfahrens werden nun näher erläutert.

### Schritt a)

Durch das erfindungsgemäße Verfahren läßt sich insbesondere IPDA gewinnen, bei dem der Anteil an Verunreinigungen weniger als 2 Gew.-%, bevorzugt weniger als 1 Gew.-%, besonders bevorzugt weniger als 0,3 Gew.-%, beträgt.

Im allgemeinen kann jedes IPDA enthaltende Produktgemisch, welches bei einem Verfahren zur Herstellung von IPDA anfällt, eingesetzt werden. Bevorzugt wird ein Produktgemisch eingesetzt, das mindestens 70 Gew.-% IPDA, bevorzugt mindestens 88 Gew.-% IPDA, besonders bevorzugt mindestens 92 Gew.-% IPDA, ganz besonders bevorzugt mindestens 95 Gew.-% IPDA, enthält.

IPDA mit einem cis/trans-Isomerenverhältnis ≤ 66/34 läßt sich nach jedem beliebigen, aus dem Stand der Technik bekannten und beispielsweise vorstehend diskutierten Verfahren zur Herstellung von IPDA aus IPN, H₂ und NH₃ in Gegenwart eines üblichen Hydrierkatalysators, wie z.B. Ra-Nickel gewinnen, insbesondere auch nach in der EP-B 0 729 937 und in der prioritätsälteren, nicht vorveröffentlichten DE-A 101 42 635 beschriebenen Verfahren.

Da IPDA mit einem cis/trans-Isomerenverhältnis von mindestens 73/27 gewonnen werden soll, ist es wirtschaftlich nur sinnvoll, Produktgemische, die IPDA mit einem cis/trans-Isomerenverhältnis kleiner als 73/27 enthalten, fraktioniert zu destillieren. Da die Herstellung von IPDA mit einem cis/trans-Isomerenverhältnis kleiner als 70/30 unter Verwendung von alternden Katalysatoren und ohne aufwendige Verfahrensdurchführung erfolgen kann, ist das erfindungsgemäße Verfahren besonders wirtschaftlich, wenn IPDA mit einem cis/trans-Isomerenverhältnis kleiner als 70/30 eingesetzt wird. Es ist auch möglich, Produktgemische einzusetzen, die IPDA mit einem cis/trans-Isomerenverhältnis größer als 73/27 enthalten, um das cis-Isomer durch fraktionierte Destillation noch weiter anzureichern.

Cis-IPDA (mit einer Reinheit von 98,9%) hat unter Normaldruck einen Siedepunkt von 253,4°C, während trans-IPDA (mit einer Reinheit von 98,4%) unter Normaldruck einen Siedepunkt von 250,7°C hat. Da also die Siedepunkte von cis- und trans-IPDA nah beieinander liegen, ist eine spezielle Verfahrensführung notwendig, um IPDA mit einem cis/trans-Isomerenverhältnis von mindestens 73/27 zu gewinnen.

### Schritte b und c)

Durch diese Schritte wird das eingesetzte, IPDA enthaltende, Produktgemisch sowohl aufgetrennt als auch gereinigt. Mögliche Komponenten/Verunreinigungen, die beispielsweise bei der Destillation des IPDA enthaltenden Produktgemisches (Roh-IPDA) abgetrennt werden, sind NH₃, sowie die bei der Herstellung von IPDA aus IPN anfallenden Nebenprodukte wie HCN-Eliminierungsprodukte, methylierte Nebenprodukte und/oder unvollständig hydrierte Zwischenprodukte.

Gemäß Schritt b) wird das IPDA zuerst in den mittleren Bereich einer (ersten) Destillationskolonne mit Einbauten geleitet. Hierfür lässt sich jede beliebige Destillationskolonne verwenden. Unter dem "mittleren Bereich" einer Destillationskolonne versteht man den Bereich zwischen Kopf und Sumpf, also den Seitenzulauf, der Destillationskolonne.

Als Einbauten sind jegliche dem Fachmann bekannte Einbauten einsetzbar. Bevorzugte Einbauten sind ausgewählt aus der Gruppe Füllkörper wie Pall-Ringe und Raschig-Ringe, strukturierte Packungen aus Blech wie Mellapak 250Y® von Sulzer Ltd. (Winterthur/Schweiz), Montz (Hilden/Deutschland) und Koch-Glitsch (Wichita, KS/USA) und strukturierte Packungen aus Metallgewebe wie Sulzer BX® von Sulzer Ltd. (Winterthur/Schweiz), Montz (Hilden/Deutschland) und Koch-Glitsch (Wichita, KS/USA).

Die Schritte b) und c) lassen sich sowohl in einer, als auch in zwei oder drei Kolonnen durchführen. Es ist auch möglich, mehr Kolonnen einzusetzen, dies ist jedoch in der Regel nicht notwendig.

Wird die fraktionierte Destillation nur in einer Kolonne durchgeführt, so wird eine Trennwandkolonne eingesetzt. Wird die fraktionierte Destillation in zwei räumlich voneinander getrennten Kolonnen durchgeführt, so ist entweder mindestens eine der beiden Kolonnen eine Trennwandkolonne oder eine der Fraktionen iii) und iv) wird an einer Seitenentnahme gewonnen.

Werden keine Trennwandkolonnen eingesetzt, so werden vorzugsweise drei herkömmliche Destillationskolonnen miteinander verschalten. Hierdurch wird eine sinnvolle Auftrennung und Reinigung erzielt.

Bevorzugt erfolgt die Auftrennung und Reinigung des IPDA in zwei Kolonnen, wovon eine eine Trennwandkolonne ist, besonders bevorzugt ist die zweite Kolonne eine Trennwandkolonne.

Werden die Schritte b) und c) in einer Trennwandkolonne durchgeführt, so werden die leicht siedenden Verunreinigungen (Fraktion i)) über Kopf der Kolonne und die schwer siedenden Verunreinigungen über Sumpf der Kolonne abgezogen. Der am Sumpf der Kolonne abgezogene Strom wird erst mit Hilfe eines Verdampfers verdampft. Die verdampfbaren Anteile werden dann in die Kolonne zurückgeführt, während die nicht verdampfbaren Anteile, bei denen es sich um die schwer siedenden Verunreinigungen handelt (Fraktion ii)), ausgeschleust werden. Entsprechend wird mit dem am Kopf der Kolonne entnommenen Strom verfahren. Dieser wird zunächst in einem Kondensator kondensiert und in einem Phasenabscheider getrennt. Die schwerere wäßrige Phase (Fraktion ib)), bei der es sich um behandlungsbedürftiges Abwasser handelt, wird ausgeschleust, während die leichtere organische Phase (Fraktion ia)) teilweise in die Kolonne zur weiteren Auftrennung rückgeführt wird. Das gewünschte Wertprodukt, die an cis-angereicherte Fraktion (Fraktion iii)) wird auf der dem Zulauf gegenüberliegenden Seite des Trennblechs im unteren Bereich der Kolonne, d. h. oberhalb des Sumpfs der Kolonne, entnommen. Die an trans-Isomer angereicherte Fraktion (Fraktion iv)) wird auf der dem Zulauf gegenüberliegenden Seite des Trennblechs im oberen Bereich der Kolonne, d. h. unterhalb des Kopfes der Kolonne, entnommen. Die Trennwandkolonne wird im allgemeinen bei Sumpftemperaturen von 170 bis 250°C, bevorzugt von 170 bis 200°C, und Kopftemperaturen von 10 bis 90°C, bevorzugt von 15 bis 65°C; betrieben. Der Druck in der Kolonne liegt im allgemeinen bei 20 bis 200 mbar, bevorzugt bei 35 bis 50 mbar.

Werden zwei Kolonnen miteinander verschalten, von denen eine eine Trennwandkolonne ist, so kann die Trennwandkolonne entweder als erste oder als zweite Kolonne eingesetzt werden. Hier werden die leicht siedenden Verunreinigungen (Fraktion i)) über Kopf der ersten und/oder der zweiten Kolonne und die schwer siedenden Verunreinigungen (Fraktion ii)) über Sumpf der ersten und/oder der zweiten Kolonne abgetrennt. Es hat sich als vorteilhaft erwiesen, die leicht siedenden Verunreinigungen über Kopf der ersten Kolonne abzutrennen.

Wie im Falle des Einsatzes von lediglich einer Kolonne wird auch hier der über Kopf abgezogene Strom erst in einem Kondensator kondensiert und dann in einem Phasenabscheider in eine schwerere wäßrige Fraktion (Fraktion ib)) und eine leichtere organische Fraktion (Fraktion ia)) getrennt. Hierbei wird die organische Fraktion (Fraktion ia)) teilweise zur weiteren Auftrennung in die Kolonne rückgeführt.

Werden auch die schwer siedenden Verunreinigungen - über Sumpf der ersten Kolonne - abgetrennt, so wird der von leicht und schwer siedenden Verunreinigungen weitgehend befreite IPDA-Strom dem mittleren Bereich der ersten Kolonne entnommen. Werden die schwer siedenden Verunreinigungen nicht über Sumpf der ersten Kolonne abgetrennt, so wird der von leicht siedenden Verunreinigungen befreite IPDA-Strom über Sumpf der ersten Kolonne ausgeschleust. Der über Sumpf der Kolonne ausgeschleuste Strom wird über einen Verdampfer geleitet und teilweise wieder in die Kolonne rückgeführt. Handelt es sich bei dem über Sumpf der Kolonne ausgeschleusten Strom um die schwer siedenden Verunreinigungen und nicht um den IPDA-Strom, so werden die nicht verdampfbaren Anteile (Fraktion ii)) ausgeschleust.

Der IPDA-Strom wird nun im mittleren Bereich in die zweite Kolonne eingeleitet. Wurden die schwer siedenden Verunreinigungen nicht bereits in der ersten Kolonne abgetrennt, so werden sie jetzt über den Sumpf der Kolonne entnommen und ausgeschleust.

Das gewünschte Wertprodukt, die an cis-Isomer angereicherte Fraktion (Fraktion iii)), wird dem Seitenablauf der zweiten Kolonne, also weder dem Kopf noch dem Sumpf, sondern dem Bereich dazwischen, entnommen. Die an trans-Isomer angereicherte Fraktion (Fraktion iv)) wird über Kopf der zweiten Kolonne abgezogen oder dem Bereich unterhalb des Kopfes, aber nicht dem Sumpf, der zweiten Kolonne entnommen.

Besonders vorteilhaft ist es, die an cis-Isomer angereicherte Fraktion (Fraktion iii)) dem unteren Bereich der zweiten Kolonne und die an trans-Isomer angereicherte Fraktion (Fraktion iv)) dem oberen Bereich der zweiten Kolonne zu entnehmen, und den über Kopf und/oder über Sumpf der zweiten Kolonne jeweils abgezogenen Strom dem in die erste Kolonne eingeschleusten Rohproduktstrom zuzumischen, um diesen Strom/diese Ströme einer erneuten Trennung zuzuführen. Die Rückführung sollte so gering wie möglich sein; üblicherweise werden etwa 1 bis 5 Gew.-% des Zulaufs rückgeführt.

Die erste Kolonne wird im allgemeinen bei Sumpftemperaturen von 150 bis 300°C, bevorzugt von 170 bis 250°C, besonders bevorzugt von 170 bis 200°C, und Kopftemperaturen von 5 bis 100°C, bevorzugt von 10 bis 90°C, besonders bevorzugt von 15 bis 65°C, betrieben. Der Druck in der ersten Kolonne liegt üblicherweise bei 10 bis 1000 mbar, bevorzugt bei 30 bis 500 mbar, besonders bevorzugt bei 35 bis 200 mbar.

Die zweite Kolonne wird im allgemeinen bei Sumpftemperaturen von 140 bis 300°C, bevorzugt von 150 bis 250°C, besonders bevorzugt von 160 bis 200°C, und Kopftemperaturen von 100 bis 250°C, bevorzugt von 130 bis 200°C, besonders bevorzugt von 140 bis 170°C, betrieben. Der Druck in der zweiten Kolonne liegt üblicherweise bei 10 bis 1000 mbar, bevorzugt bei 30 bis 300 mbar, besonders bevorzugt bei 35 bis 120 mbar.

Werden drei Destillationskolonnen miteinander verschalten, so werden die schwer siedenden Verunreinigungen jeweils über den Sumpf der ersten, zweiten und/oder dritten Kolonne abgezogen. Hierbei wird der Sumpfablauf in einem Verdampfer verdampft. Die verdampfbaren Anteile werden zur erneuten Trennung in die jeweilige Kolonne rückgeschleust, und die nicht verdampfbaren Anteile (Fraktion ii)) werden ausgeschleust. Bevorzugt erfolgt der Ablauf der schwer siedenden Verunreinigungen über den Sumpf der ersten oder der dritten Kolonne. Die leicht siedenden Verunreinigungen werden über den Kopf der ersten, zweiten und/oder dritten Kolonne abgezogen, in einem Kondensator kondensiert und anschließend in einem Phasenabscheider in eine schwerere wäßrige Fraktion (Fraktion ib)) und eine leichtere organische Fraktion (Fraktion ia)) getrennt. Die schwerere wäßrige Fraktion wird ausgeschleust, und die leichtere organische Fraktion wird teilweise der Kolonne rückgeführt bzw. teilweise ausgeschleust. Generell können sowohl zuerst die leicht siedenden Verunreinigungen als auch zuerst die schwer siedenden Verunreinigungen abgetrennt werden. Die Abtrennung der leicht und schwer siedenden Verunreinigungen erfolgt in der Regel nicht in derselben Kolonne.

Je nach Verschaltung der Kolonnen wird die an cis-Isomer angereicherte Fraktion (Fraktion iii)) entweder über Kopf oder über Sumpf der zweiten oder dritten Kolonne entnommen. Die an trans-Isomer angereicherte Fraktion (Fraktion iv)) wird ebenfalls entweder über Kopf oder Sumpf der zweiten oder dritten Kolonne entnommen. Die an cis-Isomer angereicherte und die an trans-Isomer angereicherte Fraktion können entweder derselben Kolonne, in der Regel der dritten Kolonne, oder verschiedenen Kolonnen entnommen werden. Werden die beiden Fraktionen iii) und iv) derselben Kolonne entnommen, so wird das gewünschte Wertprodukt, die an cis-Isomer angereicherte Fraktion, über den Sumpf der Kolonne, und die an trans-Isomer angereicherte Fraktion über den Kopf der Kolonne entnommen. Werden die beiden Fraktionen iii) und iv) in verschiedenen Kolonnen entnommen, so ist es möglich, beide Fraktionen über Kopf oder über Sumpf zu entnehmen. Es ist auch möglich, eine Fraktion über Kopf und die andere über Sumpf zu entnehmen.

Generell werden die über Kopf oder Sumpf der Kolonnen entnommenen Ströme in einem Kondensator kondensiert bzw. in einem Verdampfer verdampft und teilweise in die Kolonne zur weiteren Auftrennung rückgeführt.

Werden drei Kolonnen miteinander verschalten, so werden in der Regel Ströme nur über Kopf oder Sumpf der Kolonnen entnommen, und im mittleren Bereich, also dem Bereich zwischen Kopf und Sumpf, d. h. über den Seitenzulauf den einzelnen Kolonnen zugeführt.

Die drei Kolonnen werden im allgemeinen bei Sumpftemperaturen von 150 bis 250°C, bevorzugt von 170 bis 225°C, besonders bevorzugt von 170 bis 200°C, und Kopftemperaturen von 40 bis 180°C, bevorzugt von 70 bis 170°C, besonders bevorzugt von 70 bis 150°C, betrieben. Der Druck in den Kolonnen liegt üblicherweise bei 30 bis 1500 mbar, bevorzugt bei 100 bis 500 mbar, besonders bevorzugt bei 110 bis 200 mbar.

In einer Ausführungsvariante von drei miteinander verschaltenen Kolonnen werden in der ersten Kolonne die leicht siedenden Verunreinigungen (Fraktion i)) über Kopf abgezogen. Der Sumpfablauf der ersten Kolonne wird in den mittleren Bereich der zweiten Kolonne eingeleitet. Die an trans-Isomer angereicherte Fraktion (Fraktion iv)) wird über Kopf der zweiten Kolonne entnommen, während der Sumpfablauf der zweiten Kolonne zur weiteren Auftrennung in den mittleren Bereich der dritten Kolonne eingeleitet wird. Über den Sumpfablauf der dritten Kolonne werden nun die schwer siedenden Verunreinigungen (Fraktion ii)) abgetrennt, während das gewünschte Wertprodukt (Fraktion iii)) über den Kopf der dritten Kolonne entnommen wird.

In einer anderen Ausführungsvariante von drei miteinander verschaltenen Kolonnen werden in der ersten Kolonne die schwer siedenden Verunreinigungen (Fraktion ii)) über den Sumpf abgetrennt. Der über Kopf entnommene Ablauf der ersten Kolonne wird in den mittleren Bereich der zweiten Kolonne eingeleitet. Die leicht siedenden Verunreinigungen (Fraktion i)) werden über Kopf der zweiten Kolonne entnommen, während der Sumpfablauf der zweiten Kolonne zur weiteren Auftrennung in den mittleren Bereich der dritten Kolonne eingeleitet wird. Als Sumpfablauf der dritten Kolonne wird nun das gewünschte Wertprodukt (Fraktion iii)) abgetrennt, während über den Kopf der dritten Kolonne die an trans-Isomer angereicherte Fraktion (Fraktion iv)) entnommen wird.

In einer weiteren Ausführungsvariante von drei miteinander verschaltenen Kolonnen werden in der ersten Kolonne die schwer siedenden Verunreinigungen (Fraktion ii)) über den Sumpf abgetrennt. Der über Kopf entnommene Ablauf der ersten Kolonne wird in den mittleren Bereich der zweiten Kolonne eingeleitet. Die an cis-Isomer angereicherte Fraktion (Fraktion iii)) wird über den Sumpf der zweiten Kolonne entnommen, während der über Kopf der zweiten Kolonne entnommene Strom zur weiteren Auftrennung in den mittleren Bereich der dritten Kolonne eingeleitet wird. Als Sumpfablauf der dritten Kolonne wird nun die an trans-Isomer angereicherte Fraktion (Fraktion iv)) entnommen, während über den Kopf der dritten Kolonne die leicht siedenden Verunreinigungen (Fraktion i)) abgetrennt werden.

In einer vierten Ausführungsvariante von drei miteinander verschalteten Kolonnen sind die zweite und die dritte Kolonne unabhängig voneinander. Der Sumpfablauf der ersten Kolonne wird in der dritten Kolonne weiter aufgetrennt, während der über Kopf entnommene Strom in der zweiten Kolonne weiter aufgetrennt wird. Sowohl der Sumpfablauf als auch der über Kopf entnommene Strom werden jeweils in den mittleren Bereich der zweiten oder dritten Kolonne eingeleitet. Die an trans-Isomer angereicherte Fraktion (Fraktion iv)) wird über den Sumpf der zweiten Kolonne entnommen, während über den Kopf der zweiten Kolonne die leicht siedenden Verunreinigungen (Fraktion i)) abgetrennt werden. Über den Sumpfablauf der dritten Kolonne werden nun die schwer siedenden Verunreinigungen (Fraktion ii)) abgetrennt, während das gewünschte Wertprodukt (Fraktion iii)) über den Kopf der dritten Kolonne entnommen wird.

Wie bereits vorstehend bei der Beschreibung der Verschaltung der einen, zwei oder drei Kolonnen erläutert, wird durch die fraktionierte Destillation das IPDA in mindestens fünf Fraktionen -ia), ib), ii), iii) und iv) - aufgetrennt.

Die Fraktion i) welche die Teilfraktionen ia) und ib) umfaßt, enthält die leicht siedenden Komponenten/Verunreinigungen, also die Komponenten/Verunreinigungen, die niedriger sieden als trans-IPDA. Die Fraktion i) wird jeweils über Kopf der Kolonne/n abgetrennt, in einem Kondensator kondensiert und zur Trennung von organischer und wäßriger Phase in einen Phasenabscheider überführt. Die leichtere organische Phase (Fraktion ia)) wird dann entweder ganz oder teilweise zur weiteren Aufreinigung in die Kolonne rückgeführt bzw. teilweise ausgeschleust. Die schwerere wäßrige Phase (Fraktion ib)), bei der es sich um Abwasser handelt, wird einer Entsorgungsstation zugeführt. Die Menge an IPDA im Abwasser ist im allgemeinen ≤2 Gew.-%, bevorzugt ≤ 1000 ppm, besonders bevorzugt ≤ 200 ppm, jeweils bezogen auf das Gesamtgewicht des Abwassers (der Fraktion ib)).

Die Fraktion ii) enthält die schwer siedenden Komponenten/Verunreinigungen, also diejenigen Komponenten/Verunreinigungen, die höher sieden als cis-IPDA. Sie wird jeweils über den Sumpfablauf der Kolonne/n abgetrennt. Der so abgetrennte Strom wird in einen Verdampfer geleitet. Die verdampfbaren Anteile werden wieder in die Kolonne zurückgeführt, während der nicht verdampfbare Anteil ausgeschleust wird.

Die Fraktion iii) ist das gewünschte Wertprodukt. Bei ihr handelt es sich also um die an cis-IPDA angereicherte Fraktion. Die Fraktion iii) enthält IPDA mit einem cis/trans-Isomerenverhältnis ≥ 73/27, bevorzugt IPDA mit einem cis/trans-Isomerenverhältnis im Bereich von 73/27 bis 76/24, besonders bevorzugt IPDA mit einem cis/trans-Isomerenverhältnis im Bereich von 73/27 bis 75/25. Je nach Kolonne bzw. Kolonnenverschaltung wird sie entweder über Kopf, im oberen, mittleren oder unteren Bereich oder dem Sumpf der Kolonne entnommen.

Die Fraktion iv) ist die an trans-IPDA angereicherte bzw. an cis-IPDA abgereicherte Fraktion. Sie enthält im allgemeinen IPDA mit einem cis/trans-Isomerenverhältnis ≤ 66/34, bevorzugt IPDA mit einem cis/trans-Isomerenverhältnis ≤ 63/37, besonders bevorzugt IPDA mit einem cis/trans-Isomerenverhältnis ≤ 60/40. Auch sie wird - ebenso wie Fraktion iii) - entweder über Kopf, im oberen, mittleren oder unteren Bereich oder dem Sumpf der Kolonne entnommen. Auch diese Fraktion lässt sich kommerziell verwerten (siehe DE-A 42 11 454).

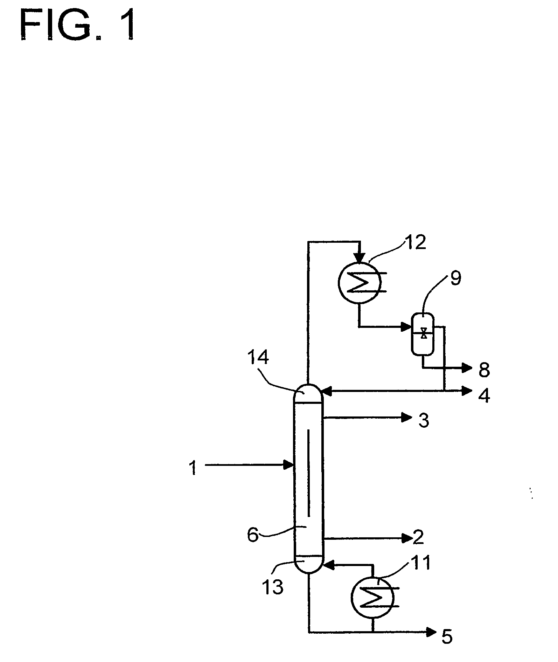

Erfindungsgemäße Ausführungsvarianten, die jedoch nicht limitierend sein sollen, werden nun anhand der dafür eingesetzten Anlagen detailliert beschrieben. Die Anlagen sind in den Figuren 1 bis 7 dargestellt.

Die Temperaturen, Drücke und theoretischen Böden der einzelnen Kolonnen werden den einzelnen Ausführungsvarianten des Verfahrens angepaßt. Generell läßt sich jedoch sagen, daß die Temperaturen und Drücke im Durchschnitt in den einzelnen Kolonnen von 5 bis 300°C bzw. von 10 bis 2000 mbar betragen und die Kolonne eine durchschnittliche Trennleistung der Böden von 20 bis 120 theoretischen Böden, bevorzugt von 25 bis 80 theoretischen Böden, besonders bevorzugt von 30 bis 60 theoretischen Böden, aufweisen.

Die anliegende Zeichnung zeigt in den Figuren 1 bis 7 schematisch Anlagen, in denen die Schritte b) und c) des erfindungsgemäßen Verfahrens zur Gewinnung von IPDA mit einem cis/trans-Isomerenverhältnis von mindestens 73/27 in einer, zwei oder drei Kolonnen durchgeführt werden, nämlich in
- **Figur 1**: eine schematische Darstellung einer Anlage, in der die einzige Kolonne eine Trennwandkolonne ist.
- **Figur 2**: eine schematische Darstellung einer Anlage, wobei die erste Kolonne eine herkömmliche Destillationskolonne und die zweite Kolonne eine Trennwandkolonne ist.
- **Figur 3**: eine schematische Darstellung einer Anlage, wobei die erste Kolonne eine Trennwandkolonne und die zweite Kolonne eine herkömmliche Destillationskolonne ist.
- **Figur 4 bis 7**: eine schematische Darstellung einer Anlage, wobei alle drei Kolonnen herkömmliche Destillationskolonnen sind.

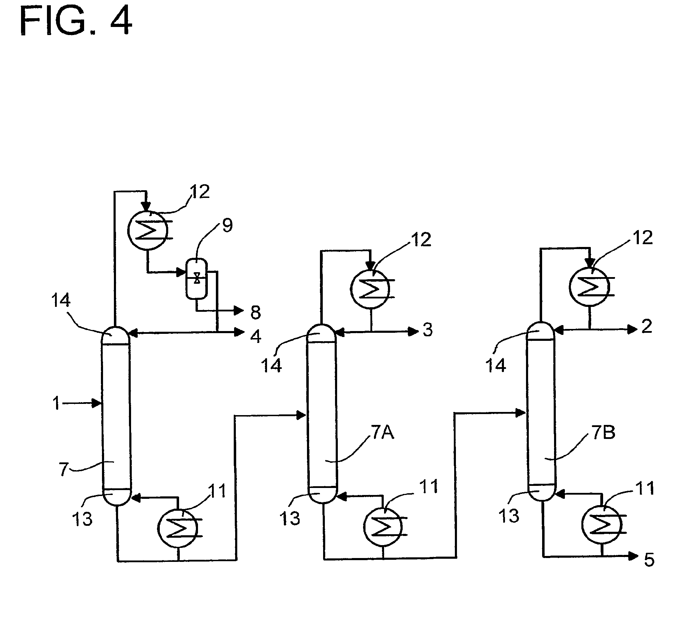

Die Anlagen gemäß der Figuren 4 bis 7 unterscheiden sich durch die Verschaltungen der Kolonnen und somit durch die Entnahmestellen der Fraktionen: Leichtsieder-Fraktion (ia) und ib)) (4), Schwersieder-Fraktion ii) (5), an cis-Isomer angereicherte IPDA-Fraktion iii) (2) und an trans-Isomer angereicherte IPDA-Fraktion iv) (3).

Wird eine Anlage gemäß Figur 1 eingesetzt, so wird die IPDA. enthaltende Produktmischung über eine Zuleitung 1 in den mittleren Bereich einer Trennwandkolonne 6 gegeben. Leicht siedende Verunreinigungen (Fraktion i)) werden über den Kopf 14 der Kolonne abgezogen, in einem Kondensator 12 kondensiert und nach Kondensation in einen Phasenscheider 9 überführt. Die leichtere organische Phase (Fraktion ia)) wird teilweise in die Kolonne 6 rückgeführt, teilweise über die Ableitung 4 ausgeschleust. Die schwerere wäßrige Phase (Fraktion ib)) wird über die Ableitung 8 abgezogen und verworfen. Schwer siedende Verunreinigungen (Fraktion ii)) werden über den Sumpf 13 der Trennwandkolonne 6 abgezogen. Ein Teil dieses Sumpfablaufs wird nach Verdampfung in einem Verdampfer 11 wieder der Kolonne 6 zugeführt, der andere Teil wird über die Ableitung 5 ausgeschleust.

Die Trennwandkolonne 6 wird im allgemeinen bei Kopftemperaturen von 10 bis 90°C, bei Sumpftemperaturen von 170 bis 250°C und/oder bei Drücken von 20 bis 200 mbar, bevorzugt bei Kopftemperaturen von 15 bis 65°C, bei Sumpftemperaturen von 170 bis 200°C und/oder bei Drücken von 35 bis 50 mbar, betrieben. Die Trennleistung beträgt im allgemeinen 1 bis 50 theoretische Böden, bevorzugt 1 bis 40 theoretische Böden, besonders bevorzugt 1 bis 35 theoretische Böden.

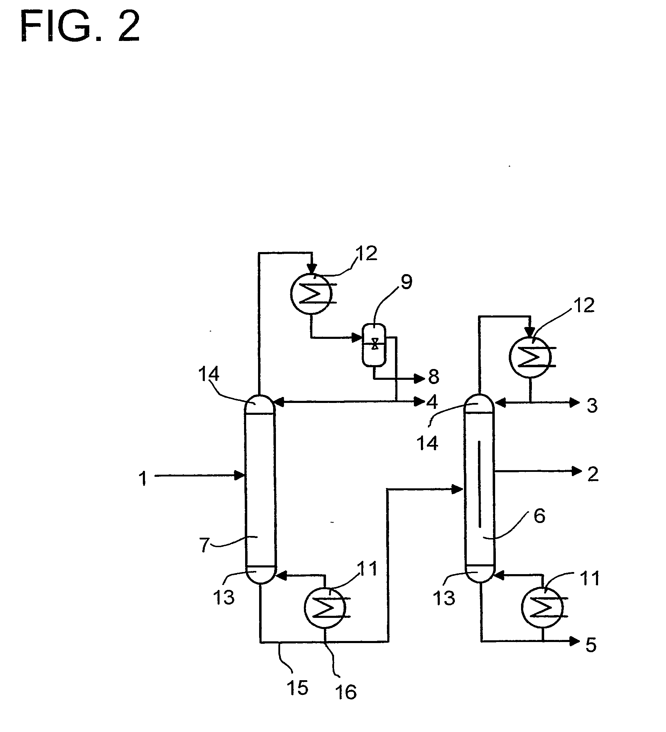

In einer Anlage gemäss Figur 2 wird ein Produktgemisch, enthaltend IPDA, über einen Einlass 1 in eine herkömmliche Destillationskolonne 7 gegeben und dort destilliert. Leichtsiedende Komponenten werden über Kopf 14 der Kolonne abgezogen, nach Kondensation in einem Kondensator 12 in einen Phasenabscheider 9 überführt und dort in eine leichtere organische und eine schwerere wäßrige Phase getrennt. Die leichtere organische Phase wird teilweise über den Abzug 4 verworfen, teilweise in die Destillationskolonne 6 rückgeführt. Die schwerere wäßrige Phase wird über die Ableitung 8 entsorgt.

Die Temperaturen am Kopf der Destillationskolonne 7 betragen im allgemeinen 20 bis 100°C, bevorzugt 30 bis 80°C und besonders bevorzugt 35 bis 65°C, die Sumpftemperaturen der Destillationskolonne 7 betragen im allgemeinen 150 bis 250°C, bevorzugt 170 bis 225°C, besonders bevorzugt 170 bis 200°C. Der durchschnittliche Druck in der Kolonne liegt bei 50 bis 1500 mbar. Bevorzugt ist ein durchschnittlicher Druck in der Kolonne von 100 bis 500 mbar, besonders bevorzugt ist ein durchschnittlicher Druck von 110 bis 200 mbar.

Der Sumpf 13 der Destillationskolonne 7 wird kontinuierlich in eine Trennwandkolonne 6 überführt. In Leitung 15 führt eine Verzweigung 16 zu einem Verdampfer 11, wo ein Teil des Sumpfablaufs wieder verdampft und in Kolonne 7 rückgeführt wird. Die an cis-Isomer angereicherte Fraktion wird über einen Seitenabzug 2 der Kolonne 6 entnommen, die an trans-Isomer angereicherte Fraktion wird über Kopf der Destillationskolonne abgezogen, in einem Kondensator 12 kondensiert und anschließend teilweise in Kolonne 6 rückgeführt, teilweise über Leitung 3 entnommen. Über den Sumpf 13 der Trennwandkolonne 6 werden hochsiedende Verunreinigungen teils über Leitung 5 ausgeschleust, teils nach Verdampfung in einem Verdampfer 11 der Kolonne 6 wieder zugeführt.

Die Temperaturen am Kopf der Trennwandkolonne 6 betragen im allgemeinen 100 bis 250°C, bevorzugt 130 bis 190°C und besonders bevorzugt 140 bis 160°C, die Sumpftemperaturen der Trennwandkolonne 6 betragen im allgemeinen 150 bis 300°C, bevorzugt 170 bis 250°C, besonders bevorzugt 170 bis 195°C. Der durchschnittliche Druck in der Kolonne liegt bei 10 bis 1000 mbar. Bevorzugt ist ein durchschnittlicher Druck in der Kolonne von 30 bis 200 mbar, besonders bevorzugt ist ein durchschnittlicher Druck von 35 bis 50 mbar.

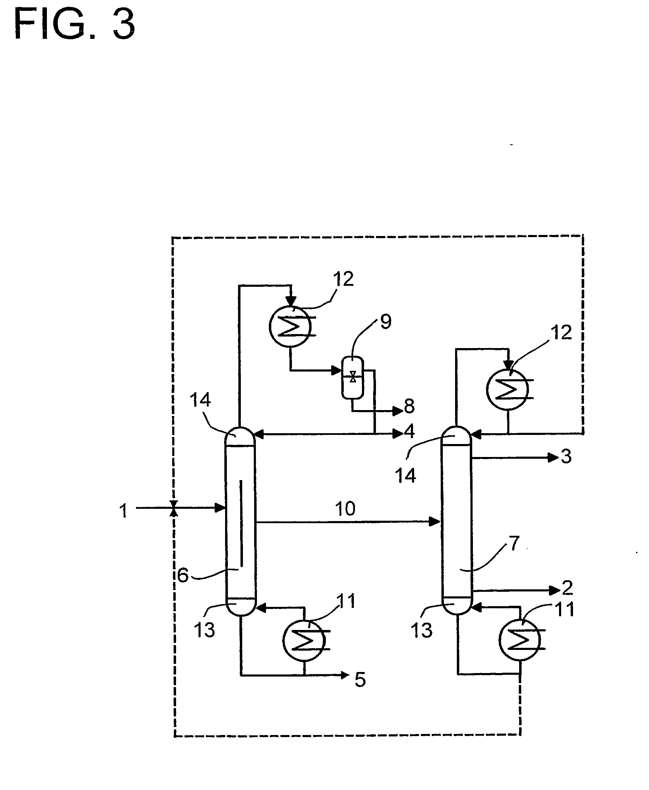

Wird Schritt b) des erfindungsgemäßen Verfahrens in einer Anlage gemäss Figur 3 durchgeführt, so wird das IPDA über die Zufuhr 1 in eine Trennwandkolonne 6 geschleust. Hochsiedende Verunreinigungen werden als Sumpf 13 der Kolonne teils über die Ableitung 5 ausgeschleust, teils nach Verdampfung in einem Verdampfer 11 der Kolonne wieder zugeführt. Leichtsiedende Verunreinigungen werden über Kopf 14 der Kolonne abgezogen und nach Kondensation in einem Kondensator 12 in einen Phasenscheider 9 überführt. Die sich abgesetzte leichtere organische Phase wird teilweise über die Ableitung 4 ausgeschleust, teilweise in die Trennwandkolonne 6 rückgeführt. Die schwerere Phase wird über die Ableitung 8 ausgeschleust.

Die Temperaturen am Kopf der Trennwandkolonne 6 betragen im allgemeinen 5 bis 100°C, bevorzugt 10 bis 90°C und besonders bevorzugt 15 bis 50°C, die Sumpftemperaturen der Trennwandkolonne 6 betragen im allgemeinen 150 bis 300°C, bevorzugt 170 bis 250°C, besonders bevorzugt 170 bis 195°C. Der durchschnittliche Druck in der Kolonne liegt bei 10 bis 1000 mbar. Bevorzugt ist ein durchschnittlicher Druck in der Kolonne von 30 bis 200 mbar, besonders bevorzugt ist ein durchschnittlicher Druck von 35 bis 50 mbar.

Das gereinigte IPDA wird über einen Seitenabzug 10 der Trennwandkolonne 6 entnommen und in eine weitere Kolonne 7 überführt, die hier als gewöhnliche Destillationskolonne ausgebildet ist.

Die Temperaturen am Kopf der Destillationskolonne 7 betragen im allgemeinen 130 bis 250°C, bevorzugt 140 bis 200°C und besonders bevorzugt 150 bis 170°C, die Sumpftemperaturen der Destillationskolonne 7 betragen im allgemeinen 140 bis 250°C, bevorzugt 150 bis 220°C, besonders bevorzugt 160 bis 190°C. Der durchschnittliche Druck in der Kolonne liegt bei 30 bis 1000 mbar. Bevorzugt ist ein durchschnittlicher Druck in der Kolonne von 50 bis 300 mbar, besonders bevorzugt ist ein durchschnittlicher Druck von 80 bis 120 mbar.

Die leichtestsiedenden Komponenten werden über Kopf 14 der Kolonne 7 abgezogen und nach Kondensation in einem Kondensator 12 teils der Kolonne 7 wieder zugeführt, teils in die Zuleitung 1 eingeschleust, um sie einer erneuten Trennung in Kolonne 6 zuzuführen. Dasselbe geschieht mit den schwerstsiedenden Komponenten, die über den Sumpf 13 der Kolonne 7 abgezogen und teilweise nach Verdampfung in einem Verdampfer 11 wieder in Kolonne 7 eingespeist, teils dem Produktgemisch in Zuleitung 1 zugegeben werden.

Die an cis-Isomer angereicherte Fraktion wird über einen Seitenabzug 2, die an trans-Isomer angereicherte Fraktion über einen Seitenabzug 3 abgezogen. Der Seitenabzug für die an cis-Isomer angereicherte Fraktion liegt unterhalb des Seitenabzugs für die an trans-Isomer angereicherte Fraktion.

Die Durchführung von Schritt b) des Verfahrens in einer Anlage gemäß Fig. 3 ist besonders vorteilhaft, da an jeweils zwei Stellen leicht und schwersiedende Verunreinigungen abgetrennt werden: Leichtsiedende Komponenten werden sowohl über Kopf 14 der Kolonne 6, als auch über Kopf 14 der Kolonne 7 abgetrennt, während schwersiedende Verunreinigungen sowohl über den Sumpfablauf 13 der Kolonne 6 als auch über den Sumpfablauf 13 der Kolonne 7 abgetrennt werden.

Bei Durchführung von Schritt b) des erfindungsgemäßen Verfahrens in Anlagen gemäss Figur 4, 5, 6 oder 7 werden jeweils drei herkömmliche Destillationkolonnen 7, 7A und 7B eingesetzt, die sich in den geometrischen Daten - dem Durchmesser und der Höhe - unterscheiden.

Die Temperaturen in den Destillationskolonnen 7, 7A und 7B betragen am Kopf im allgemeinen 40 bis 180°C, bevorzugt 70 bis 170°C, besonders bevorzugt 70 bis 150°C, die Temperaturen am Sumpf üblicherweise 150 bis 250°C, bevorzugt 170 bis 225°C, besonders bevorzugt 170 bis 190°C. Der Druck in diesen Kolonnen liegt im allgemeinen bei 30 bis 1500 mbar, bevorzugt bei 100 bis 500 mbar, besonders bevorzugt bei 110 bis 200 mbar.

Bei Durchführung des Verfahrens in einer Anlage gemäß Figur 4 wird die IPDA enthaltende Produktmischung über eine Zuleitung 1 in den mittleren Bereich der ersten Kolonne 7 gegeben. Leicht siedende Verunreinigungen werden über den Kopf der Kolonne abgezogen, in einem Kondensator 12 kondensiert und anschließend in einen Phasenscheider 9 überführt. Die leichtere organische Phase wird teilweise in die Kolonne 7 rückgeführt, teilweise über die Ableitung 4 ausgeschleust. Die schwerere wäßrige Phase wird über die Ableitung 8 abgezogen und verworfen. Das so gereinigte IPDA, welches noch hochsiedende Verunreinigungen enthalten kann, wird über den Sumpf der ersten Kolonne 7 abgezogen und in den mittleren Bereich der zweiten Kolonne 7A eingeleitet. Über den Kopf der zweiten Kolonne 7A wird jetzt die an trans-Isomer angereicherte Fraktion abgezogen, in einem Kondensator kondensiert, teilweise über die Leitung 3 abgeführt und teilweise in die Kolonne 7A rückgeführt. Über den Sumpf der Kolonne 7A wird die an cis-Isomer angereicherte Fraktion, welche noch schwer siedende Verunreinigungen enthalten kann, abgezogen und in den mittleren Bereich der dritten Kolonne 7B eingeleitet. Über den Sumpf der dritten Kolonne werden jetzt schwer siedende Verunreinigungen über die Ableitung 5 ausgeschleust. Die an cis-Isomer angereicherte Fraktion wird über Kopf der Kolonne 7B entnommen, in einem Kondensator 11 kondensiert und anschließend teilweise über die Leitung 2 entnommen, teilweise zur erneuten Trennung in Kolonne 7B zurückgeleitet.

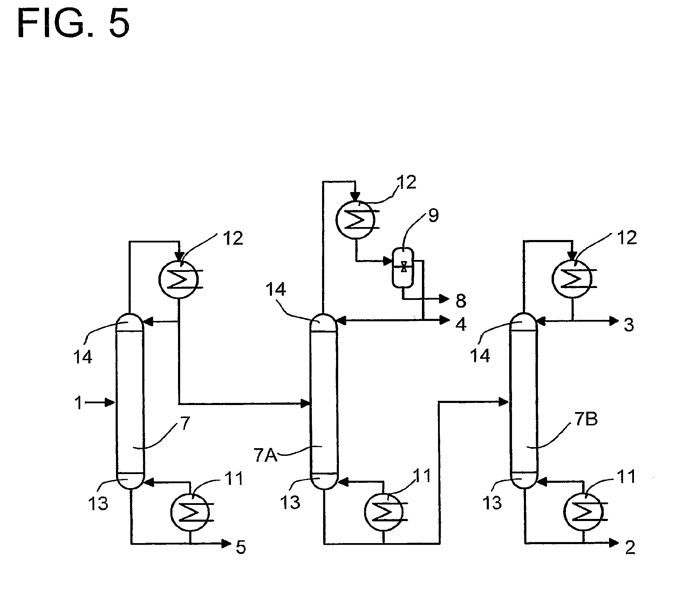

Bei Durchführung des Verfahrens in einer Anlage gemäß Figur 5 wird die IPDA enthaltende Produktmischung über eine Zuleitung 1 in den mittleren Bereich der ersten Kolonne 7 eingeschleust. Über den Sumpf dieser ersten Kolonne werden jetzt schwer siedende Verunreinigungen entnommen, in einem Kondensator 11 kondensiert und anschließend die schwerer flüchtigen Bestandteile über die Ableitung 5 ausgeschleust und die leichter flüchtigen Anteile zur erneuten Trennung in Kolonne 7 zurückgeschleust. Über den Kopf der Kolonne 7 wird die so gereinigte IPDA enthaltende Fraktion abgezogen, in einem Kondensator 11 kondensiert, teilweise in die Kolonne 7 rückgeführt und teilweise in den mittleren Bereich der zweiten Kolonne 7A eingeschleust. Über den Kopf dieser zweiten Kolonne 7A werden leicht siedende Verunreinigungen abgezogen, in einem Kondensator 11 kondensiert und anschließend in einen Phasenscheider 9 überführt. Die leichtere organische Phase wird teilweise in die Kolonne 7A rückgeführt, teilweise über die Ableitung 4 ausgeschleust. Die schwerere wäßrige Phase wird über die Ableitung 8 abgezogen und verworfen. Das so gereinigte IPDA wird nun zur weiteren Trennung von cis- und trans IPDA über den Sumpf der zweiten Kolonne 7A abgezogen und in den mittleren Bereich der dritten Kolonne 7B eingeleitet. Über den Sumpf der Kolonne 7B wird die an cis-Isomer angereicherte Fraktion abgezogen und in einem Kondensator 11 kondensiert. Die schwerer flüchtigen Bestandteile, bei denen es sich um cis-Isomer handelt, werden über die Ableitung 2 ausgeschleust, die leichter flüchtigen Bestandteile in die Kolonne 7B zur erneuten Trennung zurückgeleitet.

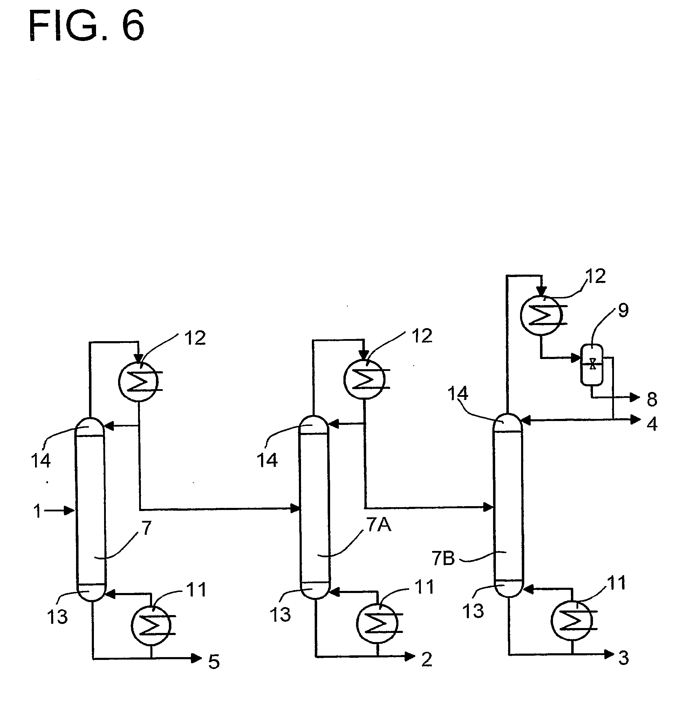

Bei Durchführung des Verfahrens in einer Anlage gemäß Figur 6 wird die IPDA enthaltende Produktmischung über eine Zuleitung 1 in den mittleren Bereich der ersten Kolonne 7 gegeben. Leicht siedende Verunreinigungen werden über den Kopf 14 der Kolonne 7 abgezogen und in einem Kondensator 12 kondensiert. Ein Teil wird anschließend in die Kolonne 7 rückgeführt, der andere Teil wird zur weiteren Trennung in den mittleren Bereich der zweiten Kolonne 7A eingeschleust. Über den Sumpf 13 der ersten Kolonne 7 wird kontinuierlich Sumpfablauf entnommen und teilweise über Leitung 5 ausgeschleust (Fraktion ii)), teilweise nach Verdampfung in dem Verdampfer 11 wieder in Kolonne 7 rückgeführt. Über den Kopf 14 der zweiten Kolonne 7A wird jetzt die an trans-Isomer angereicherte, noch leichtsiedende Komponenten/Verunreinigungen enthaltende Fraktion abgezogen, in einem Kondensator 12 kondensiert und teilweise in den mittleren Bereich der dritten Kolonne 7B eingeleitet, teilweise in die Kolonne 7A rückgeführt. Über den Sumpf 13 der Kolonne 7A wird die an cis-Isomer angereicherte Fraktion (Fraktion iii)) abgezogen oder nach Verdampfung in einem Verdampfer 11 der Kolonne 7A wieder zugeführt. Über den Kopf 14 der dritten Kolonne 7B werden jetzt leichtsiedende Verunreinigungen entnommen, in einem Kondensator 12 kondensiert und anschließend in einen Phasenabscheider gegeben. Die leichtere organische Phase (Fraktion ia)) wird teilweise über die Leitung 4 entnommen, teilweise zur erneuten Trennung in Kolonne 7B zurückgeleitet. Die schwerere wäßrige Phase (Fraktion ib)) wird über die Ableitung 8 ausgeschleust. Die an trans-Isomer angereicherte Fraktion wird über den Sumpf 13 der Kolonne 7B entnommen, teilweise in einem Verdampfer 11 verdampft und der Kolonne 7B wieder zugeführt, teilweise über die Leitung 3 ausgeschleust.

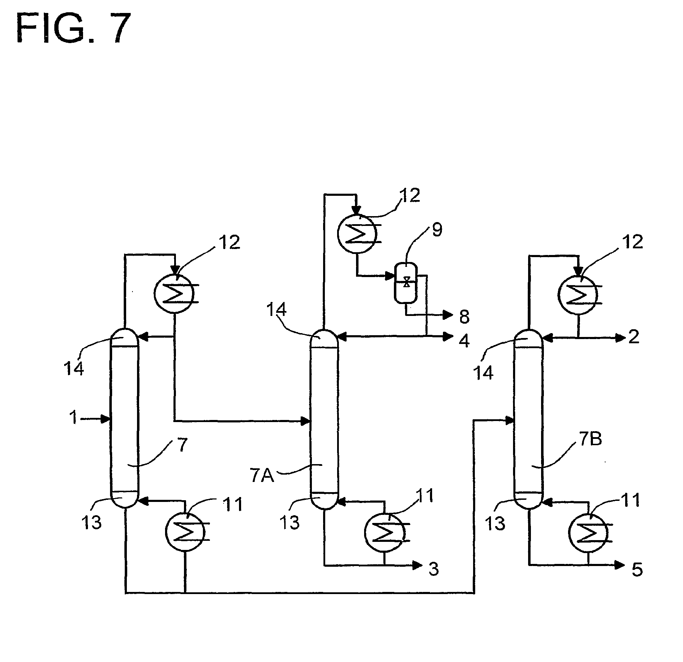

Bei Durchführung des Verfahrens in einer Anlage gemäß Figur 7 wird die IPDA enthaltende Produktmischung über eine Zuleitung 1 in den mittleren Bereich der ersten Kolonne 7 gegeben. Der leichter siedende Anteil der Produktmischung wird über den Kopf 14 der Kolonne 7 abgezogen, in einem Kondensator 12 kondensiert und anschließend teilweise in die zweite Kolonne 7A überführt, teilweise in Kolonne 7 rückgeschleust. Der schwerer siedende Anteil der Produktmischung wird über den Sumpfablauf 13 der Kolonne 7 entnommen. Er wird teilweise im Verdampfer 11 verdampft und wieder in Kolonne 7 rückgeschleust, teilweise in den mittleren Bereich der dritten Kolonne 7B eingeschleust.

In der zweiten Säule erfolgt die Trennung der Leichtsieder-Fraktion (Fraktion i)) und der an trans-Isomer angereicherten Fraktion (Fraktion iv)). Über den Kopf 14 der zweiten Kolonne 7A werden die leichtsiedenden Komponenten/Verunreinigungen (Fraktion i)) abgezogen, in einem Kondensator 12 kondensiert und in einen Phasenabscheider 9 überführt. Die leichtere organische Phase (Fraktion ia)) wird teilweise über die Leitung 4 ausgeschleust, teilweise in die Kolonne 7A rückgeführt. Die schwerere wäßrige Phase (Fraktion ib)) wird über Leitung 8 entnommen. Die an trans-Isomer angereicherte Fraktion wird über den Sumpf 13 der zweiten Kolonne 7A abgezogen, teilweise wieder verdampft in einem Verdampfer 11 und in die Kolonne 7A zurückgeschleust, teilweise über die Leitung 3 abgezogen.

In der dritten Säule erfolgt die Trennung der Hochsieder-Fraktion (Fraktion ii)) und der an cis-Isomer angereicherten Fraktion (Fraktion iii)). Über den Sumpf der dritten Kolonne werden schwer siedende Verunreinigungen über die Ableitung 5 teils ausgeschleust, teils nach Verdampfung in einem Verdampfer 11 wieder in die Kolonne 7B rückgeschleust. Die an cis-Isomer angereicherte Fraktion wird über Kopf der Kolonne 7B entnommen, in einem Kondensator 12 kondensiert und anschließend teilweise über die Leitung 2 entnommen, teilweise zur erneuten Trennung in Kolonne 7B zurückgeleitet.

Die Erfindung wird nun in den folgenden Ausführungsbeispielen zusätzlich näher erläutert.

### Ausführungsbeispiele

In den Ausführungsbeispielen wurde die Destillation von Roh-IPDA mit der in Tabelle 1 angegebenen Zusammensetzung untersucht. Die geforderten Produktreinheiten für die dadurch erhältlichen an trans-IPDA bzw. cis-IPDA angereicherten Fraktionen sind ebenfalls der Tabelle 1 zu entnehmen. Zusätzlich sollten bei der Hochsieder- und der Leichtsiederabtrennung maximal 0,5 kg/h IPDA (cis und trans) im Leichtsieder- und im Abwasserstrom und maximal 10 kg/h IPDA (cis und trans) im Hochsiederstrom verloren werden.

Alle Ausführungsbeispiele sind mit der Simulationssoftware CHEMASIM der BASF AG berechnet worden. Die Apparate sind mit Auslegungsprogrammen der BASF AG dimensioniert worden. Die Stoffdaten des betrachteten Systems sind anhand von Betriebsnachrechnungen an vorhandenen Apparaten überprüft worden.

**Tabelle 1: Zusammensetzung des zu destillierenden Roh-IPDA und geforderte Reinheit der dadurch erhältlichen Fraktionen**

| | Roh-IPDA | an trans-IPDA angereicherte Fraktion | an cis-IPDA angereicherte Fraktion |
|---|---|---|---|
| Wasser | 9,5 Gew.-% | 250 ppm | 250 ppm |
| Leichtsieder | 4,5 Gew.-% | < 250 ppm | < 250 ppm |
| trans-IPDA | 26,1 Gew.-% | ≥ 43 Gew.-% | ≤ 24 Gew.-% |
| cis-IPDA | 55,5 Gew.-% | ≤ 57 Gew.-% | ≥ 76 Gew.-% |
| Hochsieder | 4,4 Gew.-% | < 50 ppm | < 50 ppm |
| cis/trans-Isomerenverhältnis | 68/32 | 57/43 | 76/24 |
| destillierte/erhaltene Menge | 2160 kg/h | 430 kg/h | 1320 kg/h |

### Beispiel 1: Durchführung des erfindungsgemäßen Verfahrens in einer Anlage gemäss Figur 4

2160 kg/h einer IPDA enthaltenden Produktmischung werden über eine Zuleitung 1 auf den 15ten von 28 Böden der ersten Kolonne 7 gegeben. Leicht siedende Verunreinigungen werden über den Kopf der Kolonne abgezogen und in einem Kondensator 12 kondensiert, welcher bei einer Temperatur von 45°C betrieben wird. Das Kondensat wird anschließend in einen Phasenscheider 9 überführt. Die leichtere organische Phase wird teilweise in die Kolonne 7 rückgeführt, teilweise - in einer Menge von 108 kg/h - abgezogen. Die schwerere wäßrige Phase wird in einer Menge von 175 kg/h abgezogen und einer Abwasserbehandlung zugeführt. Der Anteil von IPDA in den abgezogenen organischen und wässrigen Phasen liegt bei 0,5 kg/h.

Das so gereinigte IPDA, welches < 55 ppm leichtsiedende Verunreinigungen enthält, wird über den Sumpf der ersten Kolonne 7 abgezogen und auf den 10ten von 31 Böden der zweiten Kolonne 7A eingeleitet. Über den Kopf der zweiten Kolonne 7A wird jetzt die an trans-Isomer angereicherte Fraktion abgezogen, in einem Kondensator kondensiert, teilweise - in einer Menge von 434 kg/h mit einem Gehalt an trans-IPDA von 43,2% und einem Gehalt an Leichtsiedern < 250 ppm - über die Leitung 3 abgeführt und teilweise in die Kolonne 7A rückgeführt. Über den Sumpf der Kolonne 7A wird die an cis-Isomer angereicherte Fraktion, welche noch schwer siedende Verunreinigungen enthalten kann, in einer Menge von 1421 kg/h abgezogen und auf den 10ten von 20 Böden der dritten Kolonne 7B eingeleitet. Über den Sumpf der dritten Kolonne werden jetzt schwer siedende Verunreinigungen über die Ableitung 5 in einer Menge von 105 kg/h ausgeschleust, wobei der Anteil an IPDA < 10 kg/h ist. Die an cis-Isomer angereicherte Fraktion wird über Kopf der Kolonne 7B entnommen, in einem Kondensator 11 kondensiert und anschließend teilweise über die Leitung 2 - in einer Menge von 1316 kg/h mit einem cis-Gehalt von 76% und einem Anteil von Hochsiedem < 50 ppm - entnommen, teilweise zur erneuten Trennung in Kolonne 7B zurückgeleitet.

Details zu den Temperatur- und Druckverhältnissen in den Kolonnen 7, 7A und 7B, den für die Trennung erforderlichen Rücklaufverhältnissen, sowie ihren Einbauten und Abmessungen sind Tabelle 2 zu entnehmen.

**Tabelle 2: Kenndaten der Kolonnen 7, 7A und 7B:**

| | Kolonne 7 | Kolonne 7A | Kolonne 7B |
|---|---|---|---|
| Durchmesser [mm] | 900 | 1400 | 1100 |
| Höhe [m] | 22 | 19 | 16 |
| Druck am Kopf der Kolonne [mbar] | 100 | 100 | 35 |
| Temperatur am Kopf der Kolonne [°C] | 87 | 167 | 140 |
| Temperatur am Sumpf der Kolonne [°C] | 170 | 174 | 176 |
| Rücklaufverhältnis | 3,1 | 14,9 | 1,05 |
| Wärmeleistung am Sumpf der Kolonne [kW] | 277 | 615 | 222 |
| Einbauten im Verstärkungsteil der Kolonne | 2 x 3,5 m Blechpakkung mit 250 m²/m³ | 2 x 4,0 m Blechpakkung mit 250 m²/m³ | 1 x 4,0 m Blechpakkung mit 250 m²/m³ |
| Einbauten im Abtriebsteil der Kolone | 2 x 3,5 m Blechpakkung mit 250 m²/m³ | 1 x 4,0 m Blechpakkung mit 250 m²/m³ | 1 x 4,0 m Blechpakkung mit 250 m²/m³ |

### Beispiel 2: Durchführung des erfindungsgemäßen Verfahrens in einer Anlage gemäss Figur 2

2160 kg/h eines Produktgemischs enthaltend IPDA werden über einen Einlass 1 auf den 15ten Boden von 28 Böden einer Kolonne 7 gegeben, wobei diese mit der unter Beispiel 1 beschriebenen Kolonne 7 übereinstimmt, d. h. die Kenndaten und die Betriebsbedingungen sind gleich. Leichtsiedende Komponenten werden über Kopf 14 der Kolonne 7 abgezogen, nach Kondensation in einem Kondensator 12 in einen Phasenabscheider 9 überführt und dort in eine leichtere organische und eine schwerere wäßrige Phase getrennt. Die leichtere organische Phase wird teilweise - in einer Menge von 108 kg/h - über den Abzug 4 verworfen, teilweise in die Destillationskolonne 6 rückgeführt. Die schwerere wäßrige Phase wird in einer Menge von 175 kg/h über die Ableitung 8 entsorgt, wobei der Anteil an IPDA in den abgezogenen Strömen 0,5 kg/h ist.

Der Sumpf 13 der Destillationskolonne 7 enthaltend ein Gemisch aus Hochsieder, cis- und trans-IPDA wird kontinuierlich in eine Trennwandkolonne 6 überführt und zwar auf den 15ten von 44 Böden auf der Zulaufseite. Die an cis-Isomer angereicherte Fraktion wird über einen Seitenabzug 2 der Kolonne 6 in einer Menge von 1320 kg/h mit einem cis-Gehalt von 76% und einem Gehalt an Hochsiedern unter 50 ppm entnommen, die an trans-Isomer angereicherte Fraktion wird über Kopf der Destillationskolonne abgezogen und in einem Kondensator 12 kondensiert, welcher bei 139°C betrieben wird. Das Kondensat wird anschließend teilweise in Kolonne 6 rückgeführt, teilweise über Leitung 3 in einer Menge von 430 kg/h wobei der trans-Gehalt bei 57% liegt, entnommen. Über den Sumpf 13 der Trennwandkolonne 6 werden hochsiedende Verunreinigungen teils über Leitung 5 - in einer Menge von 104 kg/h mit einem Gehalt an IPDA < 10 kg/h - ausgeschleust, teils nach Verdampfung in einem Verdampfer 11 der Kolonne 6 wieder zugeführt.

Details zu den Temperatur- und Druckverhältnissen in den Kolonnen 7 und 6, den für die Trennung erforderlichen Rücklaufverhältnissen, sowie ihren Einbauten und Abmessungen sind Tabelle 3 zu entnehmen.

**Tabelle 3: Kenndaten der Kolonnen 7 und 6 (Trennwandkolonne):**

| | Kolonne 7 | Kolonne 6 |
|---|---|---|
| Durchmesser [mm] | 900 | 1600 |
| Höhe [m] | 22 | 19 |
| Druck am Kopf der Kolonne [mbar] | 100 | 35 |
| Temperatur am Kopf der Kolonne [°C] | 87 | 139 |
| Temperatur am Sumpf der Kolonne [°C] | 170 | 185 |
| Rücklaufverhältnis | 0,46 | 13,4 |
| Wärmeleistung am Sumpf der Kolonne [kW] | 277 | 547 |
| Aufteilungsverhältnis Flüssigkeit oberhalb Trennblech; Zulaufseite : Abzugseite | | 0,436 : 1 |
| Aufteilungsverhältnis Dampf unterhalb Trennblech; Zulaufseite : Abzugseite | | 0,7 : 1 |
| Einbauten im Verstärkungsteil der Kolonne | 2 x 3,5 m Blechpackung mit 250 m²/m³ | 1 x 4,0 m Gewebepackung mit 500 m²/m³ |
| Einbauten Trennblechbereich Zulaufseite/Abzugseite oberhalb Zulauf/Ablauf | | Zulaufseite: |
| | | 1 x 1,5 m Gewebepackung mit 500 m²/m³; Ablaufseite: |
| | | 1 x 3,0 m Gewebepackung mit 500 m²/m³ |
| Einbauten Trennblechbereich Zulaufseite/Abzugseite unterhalb Zulauf/Ablauf | | Zulaufseite: |
| | | 1 x 3,75 m Gewebepackung mit 500 m²/m³; Ablaufseite: |
| | | 1 x 2,25 m Gewebepackung mit 500 m²/m³ |
| Einbauten im Abtriebsteil der Kolonne | 2 x 3,5 m Blechpackung mit 250 m²/m³ | 1 x 1,75 m Gewebepackung mit 500 m²/m³ |

### Beispiel 3: Durchführung des erfindungsgemäßen Verfahrens in einer Anlage gemäss Figur 1

Wird eine einzelne Trennwandkolonne eingesetzt, so vereint diese Kolonne die Abtrennung der Leichtsieder und des Wassers am Kopf der Kolonne mit der Abtrennung der Schwersieder am Sumpf der Kolonne und der Anreicherung des cis-Isomers. Deshalb sind auf der Abzugsseite der Trennwand zwei Seitenabzüge vorgesehen. Am oberen Seitenabzug wird die an trans-IPDA angereicherte Fraktion abgezogen, am unteren Seitenabzug die an cis-Isomer angereicherte Fraktion.

2160 kg/h einer IPDA enthaltenden Produktmischung werden über eine Zuleitung 1 auf den 15ten Boden von 75 Böden der Trennwandkolonne 6 gegeben. Leicht siedende Verunreinigungen werden über den Kopf 14 der Kolonne abgezogen und in einem Kondensator 12, welcher bei einer Temperatur von 17°C betrieben wird, kondensiert. Das Kondensat wird anschließend in einen Phasenscheider 9 überführt. Die leichtere organische Phase wird teilweise in die Kolonne 6 rückgeführt, teilweise über die Ableitung 4 - in einer Menge von 108 kg/h - ausgeschleust, wobei der Anteil an IPDA < 0,5 kg/h ist. Die schwerere wäßrige Phase wird über die Ableitung 8 in einer Menge von 175 kg/h abgezogen und der Abwasserbehandlung zugeführt. Schwer siedende Verunreinigungen werden über den Sumpf 13 der Trennwandkolonne 6 in einer Menge von 105 kg/h abgezogen, wobei der Gehalt an IPDA < 10 kg/h ist. Die an cis-Isomer angereicherte Fraktion wird in Höhe des 14ten Bodens auf der Abzugseite in einer Menge von 1320 kg/h entnommen, wobei das cis/trans-Isomerenverhältnis 76/34 ist und der Gehalt an Hochsiedem < 50 ppm. Die an trans-Isomer angereicherte Fraktion wird in einer Menge von 430 Kg/h in Höhe des 60sten Bodens auf der Abzugseite entnommen, wobei das cis/trans-Isomerenverhältnis 57/43 beträgt und der Anteil an Leichtsiedern < 250 ppm ist.

Details zu den Temperatur- und Druckverhältnissen in der Kolonne 6, den für die Trennung erforderlichen Rücklaufverhältnissen, sowie ihren Einbauten und Abmessungen sind Tabelle 4 zu entnehmen.

**Tabelle 4: Kenndaten der Kolonne 6 (Trennwandkolonne):**

| | Kolonne 6 |
|---|---|
| Durchmesser [mm] | 2100 |
| Höhe [m] | 32 |
| Druck am Kopf der Kolonne [mbar] | 20 |
| Temperatur am Kopf der Kolonne [°C] | 63 |
| Temperatur am Sumpf der Kolonne [°C] | 175 |
| Rücklaufverhältnis | 10,61 |
| Wärmeleistung am Sumpf der Kolonne [kW] | 920 |
| Aufteilungsverhältnis Flüssigkeit oberhalb Trennblech; Zulaufseite : Abzugseite | 0,4 : 1 |
| Aufteilungsverhältnis Dampf unterhalb Trennblech; Zulaufseite : Abzugseite | 1 : 1 |
| Einbauten im Verstärkungsteil der Kolonne | 1 x 2,5 m Gewebepackung mit 500 m²/m³ |
| Einbauten Trennblechbereich Zulaufsei- | Zulaufseite: |
| te/Abzugseite oberhalb Zulauf/erster Ablauf | 3 x 3,5 m Gewebepackung mit 500 m²/m³; Ablaufseite: |
| | 1 x 1,25 m Gewebepackung mit 500 m²/m³ |
| Einbauten Trennblechbereich Ablaufseite zwischen erstem und zweitem Ablauf | 2 x 4,0 m, 1 x 3,0 m Gewebepackung mit 500 m²/m³ |
| Einbauten Trennblechbereich Zulaufseite/Abzugseite unterhalb Zulauf/zweitem Ablauf | Zulaufseite: |
| | 1 x 3,75 m Gewebepackung mit 500 m²/m³; Ablaufseite: |
| | 1 x 2,0 m Gewebepackung mit 500 m²/m³ |
| Einbauten im Abtriebsteil der Kolonne | 1 x 1,5 m Gewebepackung mit 500 m²/m³ |

### Bezugszeichenliste

- 1: Zufuhr des IPDA
- 2: Abzug für die an cis-Isomer angereicherte Fraktion
- 3: Abzug für die an trans-Isomer angereicherte Fraktion
- 4: Abzug für leicht siedende Verunreinigungen (organischer Anteil)
- 5: Abzug für schwer siedende Verunreinigungen
- 6: Trennwandkolonne
- 7, 7A, 7B: Destillationskolonne
- 8: Abzug für die schwereren Bestandteile der Leichtsieder-Fraktion; Abzug für das behandlungsbedürftige Abwasser
- 9: Phasenabscheider
- 10: Seitenabzug
- 11: Verdampfer
- 12: Kondensator
- 13: Sumpf der Kolonne
- 14: Kopf der Kolonne
- 15: Leitung
- 16: Verzweigung

## Patentansprüche

1. Verfahren zur Gewinnung von 3-Aminomethyl-3,5,5-trimethylcyclohexylamin (Isophorondiamin, IPDA) mit einem cis/trans-Isomerenverhältnis von mindestens 73/27 durch fraktionierte Destillation, welches folgende Schritte enthält:
a) Bereitstellen von IPDA mit einem cis/trans-Isomerenverhältnis < 73/27;
b) Einleiten von IPDA in den mittleren Bereich einer Destillationskolonne mit Einbauten und Destillieren des IPDA in dieser Destillationskolonne bei einer Sumpftemperatur von 170 bis 250 °C und einer Kopftemperatur von 10 bis 90 °C und einem Druck von 20 bis 200 mbar;
c) gegebenenfalls weiteres Aufreinigen des durch Schritt b) erhaltenen IPDA durch Destillieren in mindestens einer weiteren Kolonne;
wobei durch die Schritte b) und c) eine Auftrennung des in Schritt a) eingesetzten IPDA in mindestens fünf Fraktionen ia) bis iv) erfolgt:
ia) der organische Anteil einer Fraktion niedriger als trans-IPDA siedender Verunreinigungen,
ib) der wäßrige Anteil einer Fraktion niedriger als trans-IPDA siedender Verunreinigungen,
ii) eine Fraktion höher als cis-IPDA siedender Verunreinigungen,
iii) eine IPDA-Fraktion mit einem cis/trans-Isomerenverhältnis ≥ 73/27 und
iv) eine IPDA-Fraktion mit einem cis/trans-Isomerenverhältnis ≤ 66/34.

2. Verfahren nach Anspruch 1, **dadurch gekennzeichnet, daß** der Anteil von cis- und trans-IPDA in der durch die Schritte b) und c) erhaltenen Fraktion ib) ≤ 2 Gew.-% - bezogen auf das Gesamtgewicht der Fraktion ib) - ist.

3. Verfahren nach Anspruch 1 oder 2, **dadurch gekennzeichnet, daß** in Schritt a) IPDA mit einem cis/trans-Isomerenverhältnis < 70/30 eingesetzt wird.

4. Verfahren nach einem der Ansprüche 1 bis 3, **dadurch gekennzeichnet, daß** die in Schritt b) eingesetzte Destillationskolonne eine Trennleistung von mindestens 20 theoretischen Böden aufweist.

5. Verfahren nach einem der Ansprüche 1 bis 4, **dadurch gekennzeichnet, daß** im Falle des Einsatzes von einer oder zwei Kolonnen mindestens eine Kolonne eine Trennwandkolonne ist

6. Verfahren nach einem der Ansprüche 1 bis 5, **dadurch gekennzeichnet, daß** zwei Kolonnen eingesetzt werden, von denen eine eine Trennwandkolonne ist.

7. Verfahren nach einem der Ansprüche 1 bis 4, **dadurch gekennzeichnet, daß** zwei Kolonnen eingesetzt werden und eine der Fraktionen iii) oder iv) an einer Seitenentnahme gewonnen wird.

8. Verfahren nach einem der Ansprüche 1 bis 4, **dadurch gekennzeichnet, daß** drei herkömmliche Destillationskolonnen miteinander verschalten werden.

9. Verfahren nach einem der Ansprüche 1 bis 8, **dadurch gekennzeichnet, daß** die Einbauten der in den Schritten b) und/oder c) eingesetzten Kolonne/n ausgewählt sind aus der Gruppe Füllkörper, strukturierte Packungen aus Blech und strukturierte Packungen aus Metallgewebe.

## Claims

1. A process for recovering 3-aminomethyl-3,5,5-trimethylcyclohexylamine (isophoronediamine, IPDA) having a cis/trans isomer ratio of at least 73/27 by fractional distillation, which comprises the following steps:
a) providing IPDA in a cis/trans isomer ratio of < 73/27;
b) feeding IPDA into the middle region of a distillation column having internals and distilling the IPDA in this distillation column at a bottom temperature of from 170 to 250°C and a top temperature of from 10 to 90°C and a pressure of from 20 to 200 mbar;
c) optionally further purifying the IPDA obtained by step b) by distilling in at least one further column;
where steps b) and c) separate the IPDA used in step a) into at least five fractions ia) to iv) :
ia) the organic proportion of a fraction of impurities having lower boiling points than trans-IPDA,
ib) the aqueous proportion of a fraction of impurities having lower boiling points than trans-IPDA,
ii) a fraction of impurities having higher boiling points than cis-IPDA,
iii) an IPDA fraction having a cis/trans isomer ratio of ≥ 73/27 and
iv) an IPDA fraction having a cis/trans isomer ratio of s 66/34.

2. The process according to claim 1, wherein the proportion of cis- and trans-IPDA in the fraction ib) obtained by steps b) and c) is ≤ 2% by weight, based on the total weight of fraction ib).

3. The process according to claim 1 or 2, wherein IPDA is used in step a) which has a cis/trans isomer ratio of < 70/30.

4. The process according to any of claims 1 to 3, wherein the distillation column used in step b) has a separating performance of at least 20 theoretical plates.

5. The process according to any of claims 1 to 4, wherein, when one or two columns are used, at least one column is a dividing wall column.

6. The process according to any of claims 1 to 5, wherein two columns are used, of which one is a dividing wall column.

7. The process according to any of claims 1 to 4, wherein two columns are used and one of the fractions iii) or iv) is removed at a sidestream takeoff.

8. The process according to any of claims 1 to 4, wherein three conventional distillation columns are connected to one another.

9. The process according to any of claims 1 to 8, wherein the internals in the column/s used in steps b) and/or c) are selected from the group of random packings, sheet metal structured packings and woven metal structured packings.

## Revendications

1. Procédé de préparation de 3-aminométhyl-3,5,5-triméthylcyclohexylamine (isophoronediamine, IPDA) avec un rapport isomérique cis/trans d'au moins 73/27, par distillation fractionnée, comportant les étapes suivantes :
a) préparation d'IPDA avec un rapport isomérique cis/trans < 73/27 ;
b) introduction de l'IPDA dans la zone médiane d'une colonne de distillation munie d'un garnissage, et distillation de l'IPDA dans cette colonne de distillation à une température de fond de colonne de 170 à 250°C, et une température de tête de 10 à 90°C, et une pression de 20 à 200 mbars ;
c) éventuellement, nettoyage complémentaire de l'IPDA obtenue à l'étape b) par distillation dans au moins une colonne complémentaire ;
dans lequel à travers les étapes b) et c), une séparation de l'IPDA utilisée à l'étape a) en au moins cinq fractions ia) à iv) intervient :
ia) la partie organique d'une fraction plus basse que celle des contaminations trans-IPDA,
ib) la partie aqueuse d'une fraction plus basse que celle des contaminations trans-IPDA,
ii) une fraction plus haute que celle des contaminations cis-IPDA,
iii) une fraction d'IPDA avec un rapport isomérique cis/trans ≥ 73/27 et
iv) une fraction d'IPDA avec un rapport isomérique cis/trans ≤ 66/34.

2. Procédé selon la revendication 1, **caractérisé en ce que** la partie de cis- et de trans-IPDA dans la fraction ib) obtenue au travers des étapes b) et c) est ≤ 2 % en poids par rapport au poids d'ensemble de la fraction ib).

3. Procédé selon la revendication 1 ou 2, **caractérisé en ce qu'**à l'étape a) de l'IPDA avec un rapport isomérique cis/trans < 70/30 est utilisée.

4. Procédé selon l'une quelconque des revendications 1 à 3, **caractérisé en ce que** la colonne de distillation utilisée à l'étape b) présente une capacité de séparation d'au moins 20 paliers théoriques.

5. Procédé selon l'une quelconque des revendications 1 à 4, **caractérisé en ce qu'**en cas d'utilisation d'une ou deux colonnes, au moins une colonne est une colonne à membrane.

6. Procédé selon l'une quelconque des revendications 1 à 5, **caractérisé en ce que** deux colonnes sont utilisées, l'une d'elles étant une colonne à membrane.

7. Procédé selon l'une quelconque des revendications 1 à 4, **caractérisé en ce que** deux colonnes sont utilisées, et une des fractions iii) ou iv) est prélevée à hauteur d'un soutirage latéral.

8. Procédé selon l'une quelconque des revendications 1 à 4, **caractérisé en ce que** trois colonnes de distillation habituelles sont interconnectées.

9. Procédé selon l'une quelconque des revendications 1 à 8, **caractérisé en ce que** les garnissages des colonnes utilisées aux étapes b) et/ou c) sont choisis dans le groupe constitué de corps pleins, garnissages structurés en tôle, et garnissages structurés en toile métallique.
